(19) Europäisches Patentamt
European Patent Office
Office européen des brevets

(11) **EP 3 280 341 B1**

(12) **EUROPEAN PATENT SPECIFICATION**

(45) Date of publication and mention
of the grant of the patent:
**29.08.2018 Bulletin 2018/35**

(21) Application number: **16718249.2**

(22) Date of filing: **08.04.2016**

(51) Int Cl.:
*A61B 18/20* *(2006.01)*   *A61B 18/00* *(2006.01)*

(86) International application number:
**PCT/EP2016/057681**

(87) International publication number:
**WO 2016/162452 (13.10.2016 Gazette 2016/41)**

(54) **A LASER SHAVING DEVICE**

LASERRASIERVORRICHTUNG

DISPOSITIF DE RASAGE LASER

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.04.2015 EP 15163121**

(43) Date of publication of application:
**14.02.2018 Bulletin 2018/07**

(73) Proprietor: **Koninklijke Philips N.V.
5656 AE Eindhoven (NL)**

(72) Inventor: **CIUHU, Calina
5656 AE Eindhoven (NL)**

(74) Representative: **Uittenbroek, Arie Leendert
Philips Intellectual Property & Standards
High Tech Campus 5
5656 AE Eindhoven (NL)**

(56) References cited:
**EP-A1- 2 656 982     WO-A1-2013/093772
WO-A1-2014/139968**

**Description**

FIELD OF THE INVENTION

The present invention relates to a laser shaving device. The present invention also relates to a method of cutting hair.

BACKGROUND OF THE INVENTION

It is known to use a laser beam to sever hair as an alternative to an arrangement of mechanical blades. Hair exposed to a laser beam will absorb energy from the laser beam and the hair will either be severed by vaporisation. The laser beam does not require a moving cutting part to be placed against the skin or hair and so the problem of cutting elements becoming blunt is eliminated.

Published patent application WO 2014/139968 A1 discloses a device using a laser beam to sever for cutting hair. This device comprises a skin contacting face that is placed against a surface of the skin. The device also has an optical system configured to direct a cutting laser beam across a cutting zone parallel to and spaced from said skin contacting face to cut hairs extending into the cutting zone. Also provided is a laser positioning mechanism which is arranged so that the distance between the cutting laser beam and the skin contacting face is changed to maintain a predetermined distance between the cutting laser beam and the surface of the skin of a user.

It is known that laser beams can cause damage and irritation to the user if the high intensity laser beam contacts the skin. It is known to deactivate the laser beam when the skin is too close to the laser beam to avoid such damage and irritation being caused. However, deactivating the laser beam when the skin is in the vicinity of the laser beam does not prevent the formation and emission of undesired gas and smoke when the hair is exposed to the laser beam to be cut.

SUMMARY OF THE INVENTION

It is an object of the invention to provide a laser shaving device which substantially alleviates or overcomes the problems mentioned above.

According to the present invention, there is provided a laser shaving device comprising a housing having a skin engaging face and a recess extending into the housing from said skin engaging face, the skin engaging face lying in or intersecting a plane that extends across the recess and being positionable against a skin surface such that the skin surface assumes a substantially domed-shape extending in a direction out of the plane of the skin engaging face into the recess, an optical system configured to direct a laser beam across said recess to cut hairs extending from said skin surface within the recess into a cutting zone of the laser beam, a skin surface sensor configured to generate a signal indicative of a distance that a skin surface has extended out of the plane of the skin engaging face into the recess, and a controller configured to activate the laser only if said distance by which a skin surface has extended out of the plane of the skin engaging face into the recess exceeds a distance between said plane of the skin engaging face and a predetermined laser beam activation threshold.

Therefore, the number of cuts made to a hair by the laser beam is minimised. By increasing the distance the skin can travel into the recess before the laser beam is activated, the distance a hair has travelled into the recess is also increased. The result is that the first cut of the hair is closer to the desired length and requires fewer cuts to reach the desired length. The fewer cuts are performed, the less smoke is produced.

The predetermined laser beam activation threshold may be between said plane of the skin engaging face and a laser beam cutting height in the recess.

Therefore, the laser beam is only activated when the skin surface lies on the plane of the skin engaging face or in the recess. This reduces the amount of time that the laser beam is activated which saves energy.

The predetermined laser beam activation threshold may be dependent on the average distance the skin surface has extended out of the plane of the skin engaging face into the recess, preferably on both the average distance and its standard deviation.

Therefore, there will always be a point at which the skin surface has extended far enough into the recess to activate the laser beam to cut hair.

The predetermined laser beam activation threshold may be greater than or equal to $\mu_{skin} - 2* \sigma_{skin}$ and less than or equal to $\mu_{skin} + 2* \sigma_{skin}$ from the plane of the skin engaging face.

The predetermined activation threshold may be in the range of 0 um to 300 um from the plane 8 that extends across the recess 6 into the recess 6.

Therefore, the number of cuts is minimised without the probability of a cut occurring decreasing to unacceptable levels.

The laser shaving device may further comprise a laser beam sensor configured to generate information indicative of the position of the laser beam in the recess relative to said plane of the skin engaging face.

The controller may be configured to deactivate the laser beam in dependence on the information generated by the skin sensor and laser beam sensor when the distance by which the skin surface has extended out of the plane of the skin engaging face into the recess exceeds a predetermined laser beam deactivation threshold.

Therefore, the laser shaving device is able to prevent irritation or damage being caused to the skin surface.

The distance between the predetermined laser beam activation threshold and the plane of the skin engaging face may be less than the distance between a predetermined laser beam deactivation threshold and the plane of the skin engaging face.

Therefore, there is a window in which the skin surface must be located in order for cutting to take place. This helps to minimise smoke production and irritation to the skin surface.

The laser shaving device may further comprise an actuator configured to act on the optical system to adjust the position of the laser beam relative to the plane that extends across the recess.

Therefore, the distance between the predetermined laser beam deactivation threshold and the plane of the skin engaging face can be made more than the distance between a predetermined laser beam activation threshold and the plane of the skin engaging face to ensure that the laser beam is activated. Furthermore, different levels of closeness may be achieved by varying the laser beam position.

The laser shaving device may further comprise a user input configured to allow the user to control the desired closeness level and the predetermined laser beam activation threshold.

Therefore, the user is able to control his own shaving ritual and vary it depending on time limits or occasions. It removes the need for multiple devices or interchangeable heads.

The laser beam may be directed across the recess parallel to and spaced from the plane of the opening in the skin contacting face.

With this arrangement, one end of the laser beam is not closer to the skin surface. Therefore, there is less chance of the skin surface exceeding the predetermined laser deactivation threshold.

The recess in the skin engaging face may be greater than or equal to 0.3 mm wide and less than or equal to 1.5 mm wide in the direction perpendicular to the direction of the laser beam.

Therefore, the skin doming height in the recess can be controlled.

According to another aspect of the present invention, there is provided a method of cutting hair with the laser shaving device comprising operating an optical system to direct a cutting section of a laser beam across a recess, parallel and spaced from a plane of a skin engaging face, to cut hairs which extend into the recess, generating information indicative of the distance between plane of the skin engaging face and a distance by which a skin surface has extended out of the plane of the skin engaging face into the recess, a controller determining the distance by which the skin surface has extended out of the plane of the skin engaging face into the recess and activating a laser beam only if said distance by which the skin surface has extended out of the plane of the skin engaging face into the recess exceeds the distance between said plane of the skin engaging face and a predetermined laser beam activation threshold.

The method of cutting hair may further comprise the controller deactivating the laser beam when the distance by which the skin surface has extended out of the plane of the skin engaging face into the recess exceeds a predetermined laser beam deactivation threshold.

The method of cutting hair may further comprise adjusting one or more elements of the optical system in dependence on the information generated by the skin surface sensor and/or the laser beam sensor to adjust the distance between the predetermined laser beam deactivation threshold and the plane of the skin engaging face, such that the laser beam is generated.

These and other aspects of the invention will be apparent from and elucidated with reference to the embodiments described hereinafter.

BRIEF DESCRIPTION OF THE DRAWINGS

Embodiments of the invention will now be described, by way of example only, with reference to the accompanying drawings, in which:

Fig. 1 shows a perspective view of a recess in a laser shaving device for cutting hair using a laser beam;
Fig. 2 shows a front view schematic diagram of the recess end of the laser shaving device of Fig. 1;
Fig. 3 shows a schematic block circuit diagram of the laser shaving device of Fig.2; and
Fig. 4 shows a schematic diagram of the distances between the thresholds in the recess end of the laser shaving device of Fig. 2.

DETAILED DESCRIPTION OF THE EMBODIMENTS

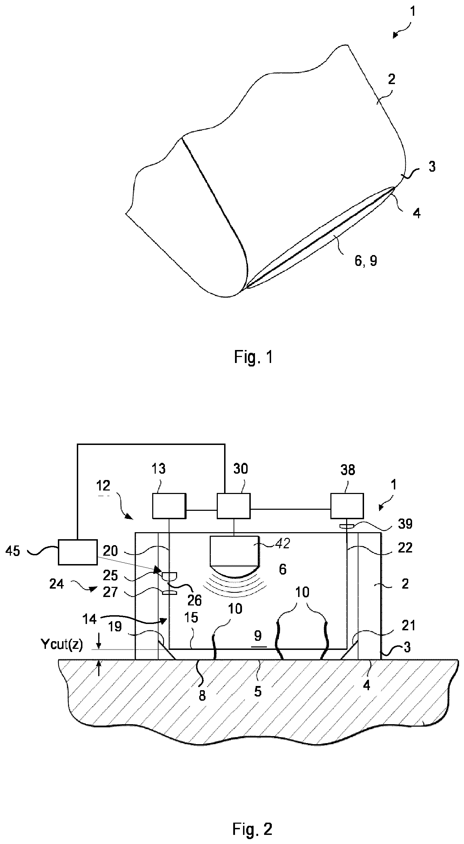

As shown in Fig. 1 and Fig. 2, a laser shaving device 1 for cutting hair comprises a housing 2. The housing 2 may comprise a guard 3. The guard 3 may be a hair and skin manipulation module. The housing 2 has a skin engaging face 4. The skin engaging face 4 is placed against a skin surface 5. The skin surface 5 may be, for example, but not limited to, the face or leg of a user or person being treated.

The skin engaging face 4 comprises a recess 6. In the present embodiment, the centre of the recess 6 is concentric with the centre of the skin engaging face 4. The recess 6 has a substantially oval cross-section. However, it will be understood that the shape of the cross-section is not limited thereto. For example, the cross-section of the recess 6 may be rectangular.

The recess 6 is a slit. The recess 6 is greater than or equal to 0.3 mm and less than or equal to 1.5 mm wide in the direction of the shaving stroke. The recess width helps to control the doming of the skin surface 5. In the present embodiment, the width of the recess 6 is 0.8 mm. The width of the recess 6 in the present embodiment helps to position the skin surface for cutting a typical 1 or 2 day beard (not shown). The length of the recess 6 in the direction perpendicular to the shaving stroke may be determined by the size of the laser shaving device 1 but preferably in the range of 10 mm to 20 mm. In the present embodiment, the length of the recess 6 is 12 mm.

In the present embodiment, the skin engaging face 4 lies in a plane 8 that extends across the recess 6. The plane 8 extends generally perpendicular to the longitudinal axis of the housing 2 of the laser shaving device 1. However, it will be understood that for ergonomic reasons the angle between the plane 8 and the longitudinal axis of the housing 2 may vary.

The recess 6 comprises a cutting zone 9. When the skin engaging face 4 of the laser shaving device 1 is placed against the skin surface 5 and moved along it, hairs 10 protruding from the skin surface 5 extend into the cutting zone 9 in the recess 6.

The housing 2 of the laser shaving device 1 comprises an optical system 12. The optical system 12 is located within the housing 2. The optical system 12 comprises a laser beam generator 13. The laser beam generator 13 may be for example, but not limited to, a laser diode. The laser beam generator 13 is configured to generate a laser beam 14. The laser beam 14 is used to cut hairs 10 which extend into the cutting zone 9 by evaporation due to optical absorption.

The optical system 12 directs the laser beam 14 across the recess 6 so that it is parallel to and spaced from the plane 8 of the recess 6 on which the skin engaging face 4 lies. The housing 2, or more specifically, the guard 3, mechanically spaces the laser beam 14 from the skin surface 5. The section of the laser beam 14 which is parallel to the skin contacting surface 4 is a cutting section 15 of the laser beam 14. The optical system 12 directs the laser beam 14 such that it extends across the recess 6 proximate to the plane 8. The point at which the cutting section 15 of the laser beam 14 travels across the recess 6 is the cutting zone 9.

In this way, when the skin engaging face 4 of the housing 2 is placed against the skin surface 5, the cutting section 15 of the laser beam 14 is substantially parallel to and spaced from the skin surface 5.

In the present embodiment, the laser beam 14 emitted by the laser beam generator 13 is initially directed downwards towards the skin surface 5. The optical system 12 redirects the laser beam 14 along the desired optical path.

The optical system 12 comprises a first reflective element 19. The first reflective element 19 is positioned on one side of the cutting zone 9. The first reflective element 19 is configured to reflect an incident section 20 of the cutting section 15 of the laser beam 14 across the cutting zone 9 of the recess 6. That is, the first reflective element 19 is configured to reflect the incident section 20 of the cutting section 15 of the laser beam 14 across the cutting zone 9, such that the cutting section 15 of the laser beam 14 follows a path which is substantially parallel to and spaced from the plane 8 that extends across the recess 6 of the laser shaving device 1.

The optical system 12 further comprises a second reflective element 21. The second reflective element 21 is positioned on an opposite side of the cutting zone 9 to the first reflective element 19. The second reflective element 21 is configured to reflect the cutting section 15 of the laser beam 14 away from the cutting zone 9. The second reflective element 21 is configured to reflect the cutting section 15 of the laser beam 14 away from the skin surface 5. The section of the laser beam 14 reflected by the second reflective element 21 is a 'used' section 22 of the laser beam 14. The 'used' section 22 of the laser beam 14 may be directed towards an energy dissipater (not shown) so that it does not interact with the skin surface 5 or another part of the laser shaving device 1.

In the embodiment shown in Fig. 2, the incident section 20 of the laser beam 14 which is incident on the first reflective element 19 is perpendicular to the plane 8 extending across the recess 6 of the housing 2. The first reflective element 19 reflects the incident section 20 of the laser beam 14 through 90 degrees such that the cutting section 15 of the laser beam 14 is parallel to the plane 8 extending across the recess 6. The second reflective element 21 is configured to reflect the cutting section 15 of the laser beam 14 through 90 degrees such that the 'used' section 22 of the laser beam 14 extends perpendicularly away from the plane 8 extending across the recess 6.

However, it will be appreciated that the first and second reflective elements 19, 21 may be orientated differently or have different reflective angles depending on the position and orientation of the other parts of the optical system 12, such as the laser beam generator 13 and the energy dissipater (not shown).

Furthermore, it will be appreciated that the first and second reflective elements 19, 21 may not be located at a side of the cutting zone 9 of Fig. 1 and Fig. 2. They may alternatively be located anywhere within the recess 6, depending on the position, orientation and configuration of the other components of the optical system 12. The first and second reflective elements 19, 21 may be omitted.

However, the cutting section 15 of the laser beam 14 should remain substantially parallel to the plane 8 extending across the recess 6 on which the skin engaging face 4 lies so that the distance between the skin engaging face 4 and the cutting section 15 of the laser beam 14 is substantially constant across the cutting zone 9 in the recess 6. Therefore, the cutting section 15 of the laser beam 14 will be prevented from irritating the skin surface 5 on one side of the cutting zone 9 and not cutting hair 10 short enough on the other.

The first and second reflective elements 19, 21 may comprise a mirror or a prism or any other optically reflective surface. Furthermore, the invention is not limited to two reflective elements.

The optical system 12 of the laser shaving device 1 further comprises a lens arrangement 24. The lens arrangement 24 is configured to focus the laser beam 14 emitted from the laser beam generator 13. In the present embodiment, as shown in Fig. 2, the lens arrangement 24 comprises a collimating lens 25. The laser beam 14 emitted from the laser beam generator 13 is directed towards the collimating lens 25. The collimating lens 25 reduces or eliminates the divergence of the laser beam 14. The collimating lens 25 of the optical system 12 produces a collimated section 26 of the laser beam 14. The collimated section 26 of the laser beam 14 is directed towards the first reflective element 19.

The lens arrangement 24 of the optical system 12 further comprises at least one focus lens 27. The focus lens 27 causes the collimated section 26 of the laser beam 14 to converge forming a converging incident section 20 of the laser beam 14. Alternatively, a plurality of focus lenses 27 can be used. The focus point of the cutting section 15 of the laser beam 14 is in the middle of the shaving width which corresponds to the axis of the recess 6. In one embodiment, the focus lenses 27 can produce a more intense collimated section 26 of the laser beam 14.

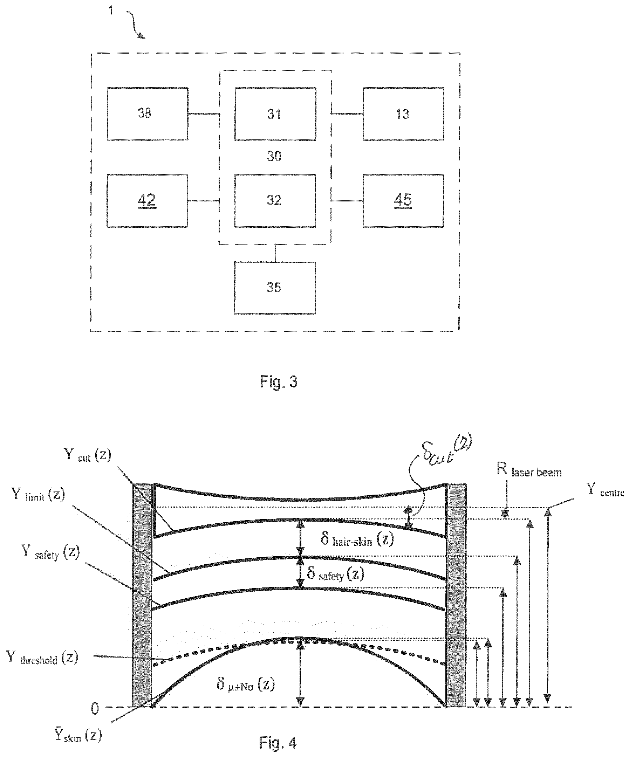

The laser shaving device 1 further comprises a controller 30. The controller 30 is configured to control the operation of the laser beam generator 13. Therefore, the controller 30 controls the operation of the cutting section 15 of the laser beam 14. Referring to Fig. 3, the controller 30 comprises a processor 31. The controller 30 further comprises a memory 32. The controller 30 is able to operate the optical system 12.

The processor 31 may take any suitable form. For instance, the processor 31 may be or include a microcontroller, plural microcontrollers, circuitry, a single processor, or plural processors. The controller 30 may be formed of one or multiple modules.

The memory 32 may take any suitable form. The memory 32 may include a non-volatile memory and/or RAM. The non-volatile memory may include read only memory (ROM), a hard disk drive (HDD) or a solid state drive (SSD). The memory stores, amongst other things, an operating system. The memory may be disposed remotely. The RAM is used by the processor 31 for the temporary storage of data.

The operating system may contain code which, when executed by the controller 30, controls the operation of each of the hardware components in the laser shaving device 1. The controller 30 may be able to cause one or more objects, such as one or more profiles, to be stored remotely or locally by the memory 32. The controller 30 may be able to refer to one or more objects, such as one or more profiles, stored by the non-volatile memory and upload the one or more stored objects to the RAM.

The controller 30 is operable to operate the laser shaving device in response to an input, for example a user input 35. The controller 30 is configured to operate the optical system 12.

The user input 35 comprises some form of user interface. Optionally, the laser shaving device 1 includes controls and/or displays for adjusting an operating characteristic of the laser shaving device 1, such as the power. The user input 35 allows a user to operate the laser shaving device 1, for example to turn the laser shaving device 1 on and off. The user input 35 may, for example, be a button, touch screen or switch.

Other components that the laser shaving device 1 may comprise, which are not shown, may include other optical components such as a filter or windows to limit the passage of detritus in the laser shaving device 1. Other components necessary for the operation of the laser shaving device 1 may also be located within the housing 2, such as a battery or a connection to an external power cable (not shown). Moreover, the housing 2 of the laser shaving device 1 may also comprise a handle and any switches, buttons or other controls and displays necessary to operate the laser shaving device 1, which may form the user input 35.

In the present embodiment, the laser shaving device 1 is provided with a laser beam sensor 38. The laser beam sensor 38 may be an electronic sensor. The laser beam sensor 38 is configured to generate information indicative of one or more optical properties of the laser beam 14 when the optical system 12 is operated. The information generated by the laser beam sensor 38 is provided to the controller 30. The controller 30 may control the operation of one or more components of the optical system 12 in dependence on the information provided by the laser beam sensor 38.

Alternatively, the laser beam sensor 38 may be a photodiode array. The laser beam sensor 38 is disposed at the end of the optical path of the laser beam 14. In the present embodiment, the laser beam sensor 38 is at the energy dissipater (not shown). However, it will be understood that alternative sensor arrangements may be used.

The laser beam sensor 38 is configured to detect one or more optical properties of the laser beam 14. A detector lens 39 is disposed on the optical path of the laser beam 14 prior to the laser beam sensor 38. The detector lens 39 is configured to adjust the dimensions of the laser beam 14 to suit the laser beam sensor 38. For example, the detector lens 39 may be configured to ensure that the dimensions of the laser beam 14 at the laser beam sensor 38 correspond to the resolution of the laser beam sensor 38. The detector lens 39 may be omitted.

The laser beam sensor 38 intersects the optical path of the laser beam 14. The laser beam sensor 38 is configured to determine one or more properties of the laser beam 14. For example, the laser beam sensor 38 may be configured to determine the position of the laser beam 14 and/or the intensity of the laser beam 14.

When the laser beam sensor 38 is configured to provide information on the position of the laser beam 14, the laser beam sensor 38 is configured to generate information on the position at which the 'used' section 22 of the laser beam 14 intersects the laser beam sensor 38. The controller 30 is able to determine the position of the cutting section 15 of the laser beam 14 relative to the plane 8 of the recess 6.

When the laser beam sensor 38 is configured to provide information on the intensity of the laser beam 14, the laser beam sensor 38 is configured to generate information on the intensity of the laser beam 14 intersecting the laser beam sensor 38. In the present arrangement that is at the end of the optical path of the laser beam 14.

In the present invention, the laser beam sensor 38 is configured to generate information indicative of the position of the laser beam 14. The controller 30 uses the information generated by the laser beam sensor 38 to determine the distance of the centre of the cutting section 15 of the laser beam 14 from the skin engaging face 4. Referring to Fig. 4, the vertical distance from the plane 8 of the recess 6 to centre of the cutting section 15 of the laser beam 14 is a centre beam height $Y_{centre}$.

The laser beam 14 has a Gaussian profile. That is, the maximum intensity of the laser beam 14 is at its centre, the centre beam height $Y_{centre}$. The controller 30 can calculate the radial variation in the intensity of the laser beam 14 based on the given power or energy of the laser beam 14, the orientation of the components of the optical system 12 and the information generated by the laser beam sensor 38. In one embodiment, the power or energy of the laser beam 14 may be controlled by the user input 35.

Due to the focusing of the laser beam 14 to increase its maximum intensity, the upper and lower edges of the laser beam 14 will vary vertically in distance from the plane 8 that extends across the recess 6 as the laser beam 14 travels across the recess 6. The lower and upper edges of the laser beam 14 are vertically above the centreline of the plane 8 that extends across the recess 6.

The edges will converge as they travel towards the focus point in the middle of the cutting zone 9 where the laser beam 14 is at its peak intensity. The edges of the laser beam 14 diverge as they travel away from the focus point. Therefore, the controller 30 can calculate the intensity and position of the cutting section 15 of the laser beam 14 at all points within the recess 6.

Alternatively, the laser beam sensor 38 may be configured to generate information indicative of both the position of the laser beam 14 and the intensity of the laser beam 14. Therefore, the controller 30 is provided information indicative of the intensity of the cutting section 15 of the laser beam 14 over a range of distances from the plane 8 of the recess 6.

In the present embodiment, the laser beam sensor 38 is configured to generate information indicative of a laser beam cutting height $Y_{cut}(z)$. The laser beam cutting height $Y_{cut}(z)$ defines the minimum distance from the plane 8 that extends across the recess 6 at which a hair 10 is cut. The laser beam cutting height $Y_{cut}(z)$ is the distance from the centreline of the plane 8 to a lower edge of a circumference defined by a radius of the cutting section 15 of the laser beam 14 which has an intensity large enough to cut hair 10.

Therefore, the cutting section 15 of the laser beam 14 comprises a cutting radius $R_{cut}(z)$. The cutting radius $R_{cut}(z)$ is defined as the difference between the centre beam distance $Y_{centre}$ and the laser beam cutting height $Y_{cut}(z)$, also shown in Fig. 4 by reference $\delta_{cut}(z)$.

Furthermore, the laser beam sensor 38 is configured to generate information indicative of an allowable intensity laser beam height $Y_{limit}(z)$. The allowable intensity laser beam height $Y_{limit}(z)$ defines the maximum distance from the plane 8 that extends across the recess 6 at which the skin surface 5 will not be harmed by the laser beam 14. The allowable intensity laser beam height $Y_{limit}(z)$ is the distance from the centreline of the plane 8 to a lower edge of a circumference defined by a radius of the cutting section 15 of the laser beam 14 which has a maximum allowable intensity without causing damage to the skin surface 5.

Therefore, the cutting section 15 of the laser beam 14 further comprises an allowable intensity radius $R_{limit}(z)$. The allowable intensity radius $R_{limit}(z)$ is defined as the difference between the centre beam distance $Y_{centre}$ and the allowable intensity laser beam height $Y_{limit}(z)$. The distance between the laser beam cutting height $Y_{cut}(z)$ and the allowable intensity laser beam height $Y_{limit}(z)$ is the minimum laser-skin distance $\delta_{laser-skin}(z)$.

The laser beam sensor 38 is configured to provide information indicative of the distance between the various heights of parts of the cutting section 15 of the laser beam 14 and the reference position. In the present embodiment, the reference position is based on the plane 8 that extends across the recess 6 of the laser shaving device 1, especially the centreline of the plane 8 which is vertically below the lowest edges of the various parts of the cutting section 15 of the laser beam 14. Therefore, the provided information indicative of the various heights of the cutting section 15 of the laser beam 14 and the reference position are the perpendicular distances. However, it will be understood that an alternative reference position may be used. For example, the reference position may be a neutral calibrated position of the cutting section 15 of the laser beam 14.

The laser shaving device 1 further comprises a skin surface sensor 42. The skin surface sensor 42 is configured to generate information indicative of the distance between the skin surface 5 in the recess 6 and the plane 8 that extends across the recess 6 when the laser shaving device 1 is positioned against the skin surface 5.

The distance measured by the skin surface sensor 42 is the skin height. However, although the skin surface 5 is in contact with the skin contacting surface 4, the skin surface 5 proximate the plane 8 that extends across the recess 6 extends into the recess 6. This is known as skin doming. Therefore, the skin surface sensor 42 measures a skin doming height $\overline{Y}_{skin}(z)$. Skin doming is exaggerated when the skin engaging face 4 is pushed into the skin surface 5 and/or the laser shaving device 1 is dragged across the skin surface 5. Because the skin surface 5 is constrained by the skin engaging face 4, the skin doming is usually largest towards the centre of the recess 6. Therefore, the skin doming height $\overline{Y}_{skin}(z)$ usually has a Gaussian profile. The information generated by the skin surface sensor 42 indicative of the skin doming distance is provided to the controller 30 which controls operation of the laser beam generator 13.

The skin surface sensor 42 may be, for example, an electronic sensor which is configured to generate information indicative of the distance between the skin surface 5 in the recess 6 and the plane 8 that extends across the recess 6 when the laser shaving device 1 is positioned against the skin surface 5.

The skin surface sensor 42 may be an optical sensor, such as a confocal lens which uses optical measuring techniques and does not need to contact the skin surface 5 to measure the distance between the skin surface 5 and the plane 8 that extends across the recess 6 of the laser shaving device 1. The skin surface sensor 42 may be configured to generate information indicative of the position of the skin surface 5 by, for example, triangulation measurement, scattered light measurement and/or shadow measurement.

The provided information indicative of the distance between the skin surface 5 in the recess 6 and the plane 8 that extends across the recess 6 is the perpendicular distance. However, it will be understood that an alternative reference position may be used. For example, the reference position may be a neutral calibrated position of the cutting section 15 of the laser beam 14.

The skin surface sensor 42 is disposed in the recess 6 of the housing 2. The skin surface sensor 42 is a non-contact skin distance sensor. That is, the skin surface sensor 42 is configured to measure the distance between the skin surface 5 in the recess 6 and the plane 8 that extends across the recess 6, when the laser shaving device 1 is positioned against the skin surface 5. The skin surface sensor 42 may be a reflective or transmissive optical sensor. The skin surface sensor 42 may use one or more wavelengths of light in the visible and/or near infra-red radiation regions. However, it will be understood that alternative sensor arrangements may be used.

The skin surface sensor 42 comprises a light source (not shown) and a detector (not shown). A light path is defined between the light source and the detector. The skin surface 5 forms part of the light path when it is disposed at the plane 8 that extends across the recess 6. When the skin surface 5 is disposed at the plane 8 that extends across the recess 6, the recess 6 is enclosed.

Although in the present arrangement the skin surface sensor 42 is disposed in the recess 6 and is configured to detect the skin surface 5 positioned relative to the plane 8 that extends across the recess 6, it will be understood that in an alternative embodiment the skin surface sensor 42 may be disposed in another position.

The controller 30 is configured to refer to the information generated by the laser beam sensor 38 indicative of the distance between the allowable intensity radius $R_{limit}(z)$ of the cutting section 15 of the laser beam 14 and the plane 8 that extends across the recess 6. The controller 30 is configured to refer to the information generated by the skin surface sensor 42 indicative of the skin doming distance $\overline{Y}_{skin}(z)$, i.e. the distance between skin surface 5 in the recess 6 and the plane 8 that extends across the recess 6 when the laser shaving device 1 is positioned against the skin surface 5. In the present embodiment, the reference positions are the same. However, in an alternative embodiment, the reference positions may be different with a known spacing.

Based on the information generated by the laser beam sensor 38 and the skin surface sensor 42, the controller 30 is able to generate information indicative of the distance between the allowable intensity radius $R_{limit}(z)$ of the cutting section 15 of the laser beam 14 and the skin doming of the skin surface 5 across the width of the recess 6.

However, due to the natural variation of the skin surface 5 which has domed into the recess 6 and laser beam instability, the allowable intensity laser beam height $Y_{limit}(z)$ can vary. This results in a range of uncertainty in which the exact position of the allowable intensity radius $R_{limit}(z)$ from the centreline $Y_{centre}$ of the laser beam 14 can not be determined.

Therefore, in order to prevent the skin surface 5 from becoming irritated by the high intensity laser beam 14 above the varying allowable intensity laser beam distance $Y_{limit}(z)$, a laser beam safety height $Y_{safety}(z)$ is determined by the controller 30. The controller 30 determines the laser beam safety height $Y_{safety}(z)$ by subtracting a safety distance $\delta_{safety}(z)$ from the allowable intensity laser beam height $Y_{limit}(z)$. The safety distance $\delta_{safety}(z)$ may be, for example, but not limited to, a percentage of the allowable intensity laser beam radius $R_{limit}(z)$ or an arbitrary distance.

The controller 30 monitors the information generated by the skin surface sensor 42 indicative of the skin doming height $\overline{Y}_{skin}(z)$ and compares it to the calculated laser beam safety height $Y_{safety}(z)$. In the event that the skin doming height $\overline{Y}_{skin}(z)$ exceeds the laser beam safety height $Y_{safety}(z)$, the controller 30 is configured to deactivate the laser beam generator 13. This removes the chance of the skin surface 5 being damaged by a part of the cutting section 15 of the laser beam 14 with too high an intensity.

Furthermore, based on the information generated by the laser beam sensor 38 and the skin surface sensor 42, the controller 30 generates a predetermined laser beam activation threshold height $Y_{threshold}(z)$. The predetermined laser beam activation threshold height $Y_{threshold}(z)$ is a distance from the plane 8 that extends across the recess 6 into the recess 6. The laser beam activation threshold height $Y_{threshold}(z)$ is the height which the skin surface 5 must reach or exceed for the laser beam 14 to be activated. Therefore, the skin doming height $\overline{Y}_{skin}(z)$ must be greater than or equal to the laser beam activation threshold height $Y_{threshold}(z)$ for the laser to be activated. As a result, the laser beam generator 13 is not activated by the controller 30 to generate the laser beam 14 unless the skin surface 5 domes into the recess 6 sufficiently. The laser beam activation threshold height $Y_{threshold}(z)$ is between the plane 8 that extends across the recess 6 and the laser beam cutting height $Y_{cut}(z)$ in the recess 6.

The laser beam activation threshold height $Y_{threshold}(z)$ is dependent on the skin doming height $\overline{Y}_{skin}(z)$. The laser beam activation threshold distance $\delta_{threshold}(z)$ may be based on the average skin doming height $\mu_{skin}$. The laser beam activation threshold distance $\delta_{threshold}(z)$ is preferably based on the average skin doming height $\mu_{skin}$ and a standard deviation of the skin doming height $\sigma_{skin}$. The laser beam activation threshold distance $\delta_{threshold}(z)$ may be based on the average skin doming height $\mu_{skin}$ and any multiple of the standard deviation $\sigma_{skin}$. The guard 3 and recess 6 can control the average skin doming height $\mu_{skin}$ and standard deviation of the skin doming height $\sigma_{skin}$. The average skin doming height $\mu_{skin}$ may be kept below 100 um. The standard deviation of the skin doming height $\sigma_{skin}$ may be controlled within 20 to 50 um.

The controller 30 is configured to determine the average skin doming height $\mu_{skin}$ and a standard deviation of the skin doming height $\sigma_{skin}$ based on information generated by the skin surface sensor 42 indicative of the position of the skin surface 5 within the recess 6, i.e. the skin doming height $\overline{Y}_{skin}(z)$ across the recess 6.

In the present embodiment, the laser beam activation threshold distance $\delta_{threshold}(z)$ is equal to $\mu_{skin} + 2* \sigma_{skin}$. However, the laser beam activation threshold distance $\delta_{threshold}(z)$ may be in the range of the plane 8 that extends across the recess 6 to the maximum skin doming height $\overline{Y}_{skin\_max}(z)$. Preferably, the laser beam activation threshold distance $\delta_{threshold}(z)$ may be in the range of $\mu_{skin} - 2* \sigma_{skin}$ to $\mu_{skin} + 2* \sigma_{skin}$. Furthermore, it will be appreciated that the laser beam activation threshold distance $\delta_{threshold}(z)$ may include an error distance $\varepsilon$. The error distance $\varepsilon$ may account for potential errors in the determination of the laser beam position and/or the skin doming height $\overline{Y}_{skin}(z)$.

The laser beam activation threshold height $Y_{threshold}(z)$ should not exceed the laser beam safety height $Y_{safety}(z)$. The controller 30 calculates the laser beam activation threshold height $Y_{threshold}(z)$. The controller 30 monitors the information generated by the skin surface sensor 42 indicative of the skin doming height $\overline{Y}_{skin}(z)$ and compares it to the calculated laser beam activation threshold height $Y_{threshold}(z)$.

In the event that the skin doming height $\overline{Y}_{skin}(z)$ equals or exceeds the laser beam activation threshold height $Y_{threshold}(z)$, the controller 30 is configured to activate the laser beam generator 13. This means that the laser beam 14 is not generated when the skin surface 5 is to far away from the laser beam 14. This results in the laser shaving device 1 using less energy to cut hair 10 and cutting each hair 10 a fewer number of times to reduce the amount of smoke created during shaving.

By imposing a laser beam activation threshold height $Y_{threshold}(z)$ and varying its distance $\delta_{threshold}(z)$ from the laser beam safety height $Y_{safety}(z)$ an average closeness for the length of hair 10 can be achieved whilst minimising the number of cuts made to a single hair 10.

The closeness of a single hair 10 is governed by the distance between the laser beam cutting height $Y_{cut}(z)$ and skin doming height $\overline{Y}_{skin}(z)$. The closer the two heights, the shorter the cut hair length $L_{stubble\ cut}$. The cut hair length is also dependent on the angle, $\cos \theta$, the hair extends into the recess 6 at relative to the vertical and the hair lift $\delta_{lift}$. For simplicity of the description both $\theta$ and $\delta_{lift}$ are assumed to be zero.

$$L_{\text{Stubble}}(z) = ((Y_{\text{cut}}(z) - \bar{Y}_{\text{skin}}(z)) / \cos \theta) - \delta_{\text{lift}}$$

if $\bar{Y}_{\text{skin}}(z) < Y_{\text{limit}}(z) - \delta_{\text{safety}}(z)$, $\bar{Y}_{\text{skin}}(z) > Y_{\text{threshold}}(z)$
and $Y_{\text{top}} > Y_{\text{cut}}(z)$

The hair 10 will only be cut when the skin doming height $\bar{Y}_{\text{skin}}(z)$ does not exceed the laser beam safety height $Y_{\text{safety}}(z)$, the skin doming height $\bar{Y}_{\text{skin}}(z)$ equals or exceeds the laser beam activation threshold height $Y_{\text{threshold}}(z)$, and the hair 10 extends further into the recess than the laser beam cutting height $Y_{\text{cut}}(z)$.

Knowing the calculation for the length $L_{\text{Stubble}}(z)$ of a hair 10 and assuming that the skin doming height $\bar{Y}_{\text{skin}}(z)$ is described by a Gaussian distribution, the length $L_{\text{Stubble}}(z)$ of a hair 10 can be given by the laser beam cutting height $Y_{\text{cut}}(z)$ minus the height y of the skin surface 5 at a particular point when the laser beam 14 is on and the original length $L_{\text{Uncut}}$ when the laser beam is off. Furthermore, the probability of each outcome can be determined.

$$L_{\text{Stubble}} = p_s(y < Y_{\text{safety}}(z)) \, ((Y_{\text{cut}}(z) - y) + p_s(y > Y_{\text{safety}}(z) \, || \, y < Y_{\text{threshold}}(z)) \, (L_{\text{Uncut}})$$

This can be applied to a stroke of the laser shaving device 1 along the skin surface 5 and an expression for the average length of the hair $L_{\text{Av}}$ stubble can be obtained.

$$L_{\text{Av stubble}} = p_s(y < Y_{\text{safety}}(z)) \, ((Y_{\text{cut}}(z) - \mu_{\text{skin}}) + p_s(y > Y_{\text{safety}}(z) \, || \, y < Y_{\text{threshold}}(z)) \, (L_{\text{Uncut}})$$

Therefore, for a desired average hair length $L_{\text{Av}}$ stubble an optimised threshold distance $\delta_{\text{threshold}}(z)$ can be calculated, dependent on the intensity of the laser beam 14 and arrangement of the optical system 12, to give the average desired hair length whilst cutting the hairs 10 fewer times.

A larger laser beam activation threshold distance $\delta_{\text{threshold}}(z)$, increases the laser beam activation threshold height $Y_{\text{threshold}}(z)$. Therefore, hair 10 is not cut until the skin surface 5 is further inside the recess 6. This means that more of the hair 10 has passed the laser beam cutting height $Y_{\text{cut}}(z)$ before the laser beam 14 is activated by the controller 30, thereby reducing the number of cuts to that hair 10.

However, the larger laser beam activation threshold height $Y_{\text{threshold}}(z)$ reduces the activation window as it is closer to the laser beam safety height $Y_{\text{safety}}(z)$, which will cause the controller 30 to deactivate the laser beam 14 if the skin surface exceeds it. This reduces the probability of the hair 10 being cut. If the laser beam activation threshold height $Y_{\text{threshold}}(z)$ becomes to great then the cutting probability becomes unacceptable for a normal shaving ritual. A cutting probability may be regarded as unacceptable if the probability falls below 15%

Conversely, the smaller the laser beam activation threshold distance $\delta_{\text{threshold}}(z)$, the further away the skin surface 5 is from the laser beam safety height $Y_{\text{safety}}(z)$ when the laser beam 14 is activated. Therefore, for a laser beam activation threshold distance $\delta_{\text{threshold}}(z)$ of less than $\mu_{\text{skin}} - 2^* \sigma_{\text{skin}}$, the probability of cutting is high for a shaving ritual. However, for this laser beam activation threshold distance $\delta_{\text{threshold}}(z)$ the hairs 10 will be cut the most number of times. The extra number of cuts to the hair 10 causes unwanted and unnecessary smoke, whilst at the same time causing the laser shaving device 1 to use more energy than necessary.

The larger the laser beam activation threshold distance $\delta_{\text{threshold}}(z)$, the closer the skin surface 5 is to the laser beam safety height $Y_{\text{safety}}(z)$ which means that the laser beam 14 is more likely to be deactivated again to protect the skin surface 5. Therefore, for a laser beam activation threshold distance $\delta_{\text{threshold}}(z)$ of $\mu_{\text{skin}} + 2^* \sigma_{\text{skin}}$, the probability of cutting is low, however, the hairs 10 will be cut fewer times, which may be acceptable for a shaving ritual.

In the present embodiment, the laser beam activation threshold $Y_{\text{threshold}}(z)$ is located within recess 6. The laser beam activation threshold $Y_{\text{threshold}}(z)$ may be located anywhere from the plane 8 that extends across the recess 6 to the maximum skin doming height. Therefore, the laser beam activation threshold height $Y_{\text{threshold}}(z)$ is preferably in the range of 0 um to 300 um from the plane 8 that extends across the recess 6 into the recess 6.

The laser shaving device 1 further comprises an actuator 45. The actuator 45 is configured to act on one or more components of the optical system 12. The actuator 45 may be configured to rotate the at least one lens 24 or the first reflective element 19 to change the centre beam height $Y_{\text{centre}}$ or intensity of the cutting section 15 of the laser beam 14.

The actuator 45 may be an electronic actuator, for example a voice coil actuator, spindle motor with gear or a piezo electrical translator. The actuator 45 acts on one or more components of the optical system 12 based on commands from the controller 30.

In the event that the laser beam activation threshold height $Y_{\text{threshold}}(z)$ exceeds the laser beam safety height

$Y_{safety}(z)$ when the skin surface 5 is in contact with the skin engaging face 4, the laser beam 14 would never be activated. Therefore, the controller 30, based on information generated by the laser beam sensor 38 and skin surface sensor 42, can command the actuator 45 to adjust one or more components of the optical system 12 so that the centre beam height $Y_{centre}$ of the cutting section 15 of the laser beam 14 is increased.

This moves the laser beam safety height $Y_{safety}(z)$ away from the laser beam activation threshold height $Y_{threshold}(z)$ and the skin doming height $\overline{Y}_{skin}(z)$. Once the skin doming height $\overline{Y}_{skin}(z)$ is below the laser beam safety height $Y_{safety}(z)$, the controller 30 can command the actuator 45 to stop adjusting one or more components of the optical system 12.

Furthermore, the user may desire a hair length that is much longer than the determined laser beam activation threshold distance $\delta_{threshold}(z)$ for a close shave whilst minimising the number of cuts to the hair. In this event, the user inputs a desired hair length using the user input 35 and the controller 30 commands the actuator 45 to adjust one or more components of the optical system 12 so that the centre beam height $Y_{centre}$ of the cutting section 15 of the laser beam 14 is adjusted to the desired distance from the skin surface 5.

Conversely, the actuator 45 may move the centre beam height $Y_{centre}$ of the cutting section 15 of the laser beam 14 away from the plane 8 that extends across the recess 6. The adjustment effected by the actuator 45 will depend on the selected variables of the laser shaving device 1.

In one embodiment, the laser beam activation threshold height $Y_{threshold}(z)$ may be the plane 8 that extends across the recess 6 or proximate thereto. In another embodiment of the invention, the controller 30 may not be configured to deactivate the laser beam generator 13 when the skin doming height $\overline{Y}_{skin}(z)$ exceeds the laser beam safety height $Y_{safety}(z)$.

It will be appreciated that the term "comprising" does not exclude other elements or steps and that the indefinite article "a" or "an" does not exclude a plurality. A single processor may fulfil the functions of several items recited in the claims. The mere fact that certain measures are recited in mutually different dependent claims does not indicate that a combination of these measures cannot be used to an advantage. Any reference signs in the claims should not be construed as limiting the scope of the claims.

**Claims**

1. A laser shaving device (1) comprising
   a housing (2) having a skin engaging face (4) and a recess (6) extending into the housing from said skin engaging face, the skin engaging face lying in or intersecting a plane (8) that extends across the recess and being positionable against a skin surface (5) such that the skin surface assumes a substantially domed-shape extending in a direction out of the plane of the skin engaging face into the recess,
   an optical system (12) configured to direct a laser beam (14) across said recess to cut hairs (10) extending from said skin surface within the recess into a cutting section (15) of the laser beam,
   a skin surface sensor (42) configured to generate a signal indicative of a distance ($\overline{Y}_{skin}(z)$) that a skin surface has extended out of the plane of the skin engaging face into the recess, and
   a controller (30) configured to activate the laser beam (14) only if said distance by which a skin surface has extended out of the plane of the skin engaging face into the recess exceeds a distance between said plane of the skin engaging face and a predetermined laser beam activation threshold ($Y_{threshold}(z)$).

2. The laser shaving device (1) according to claim 1, wherein the predetermined laser beam activation threshold ($Y_{threshold}(z)$) is between said plane (8) of the skin engaging face (4) and a laser beam cutting height ($Y_{cut}(z)$) in the recess (6).

3. The laser shaving device (1) according to claim 2, wherein the predetermined laser beam activation threshold ($Y_{threshold}(z)$) is dependent on the average distance ($\mu_{skin}$) the skin surface has extended out of the plane (8) of the skin engaging face (4) into the recess (6), preferably on both the average distance ($\mu_{skin}$) and its standard deviation ($\sigma_{skin}$).

4. The laser shaving device (1) according to claim 3, wherein the predetermined laser beam activation threshold ($Y_{threshold}(z)$) is greater than or equal to $\mu_{skin} - 2* \sigma_{skin}$ and less than or equal to $\mu_{skin} + 2* \sigma_{skin}$ from the plane (8) of the skin engaging face (4).

5. The laser shaving device (1) according to claim 2, wherein the predetermined activation threshold ($Y_{threshold}(z)$) is in the range of 0 um to 300 um from the plane (8) that extends across the recess (6) into the recess (6).

6. The laser shaving device (1) according to any one of the preceding claims, further comprising a laser beam sensor (38) configured to generate information indicative of the position of the laser beam in the recess relative to said plane (8) of the skin engaging face (4).

7. The laser shaving device (1) according to claim 6, wherein the controller (30) is configured to deactivate the laser beam (14) in dependence on the information generated by the skin surface sensor (42) and laser beam sensor (38) when the distance ($\overline{Y}_{skin}(z)$) by which the skin surface (5) has extended out of the plane (8) of the skin engaging face (4) into the recess (6) exceeds a predetermined laser beam deactivation threshold ($Y_{safety}(z)$).

8. The laser shaving device (1) according to any one of the preceding claims, wherein the distance between the predetermined laser beam activation threshold ($Y_{threshold}(z)$) and the plane (8) of the skin engaging face (4) is less than the distance between a predetermined laser beam deactivation threshold ($Y_{safety}(z)$) and the plane of the skin engaging face.

9. The laser shaving device (1) according to any one of the preceding claims, further comprising an actuator (45) configured to act on the optical system (12) to adjust the position of the laser beam (14) relative to the plane (8) that extends across the recess (6).

10. The laser shaving device (1) according to any one of the preceding claims, further comprising a user input (35) configured to allow the user to control the desired closeness level and/or the predetermined laser beam activation threshold ($Y_{threshold}(z)$).

11. The laser shaving device (1) according to any one of the preceding claims, wherein the laser beam (14) is directed across the recess (6) parallel to and spaced from the plane (8) that the skin engaging face (8) lies in that extends across the recess (6).

12. The laser shaving device (1) according to any one of the preceding claims, wherein the recess (6) in the skin engaging face (4) is greater than or equal to 0.3 mm and less than or equal to 1.5 mm wide in the direction perpendicular to the direction of the laser beam (14).

13. A method of cutting hair with a laser shaving device (1) comprising
operating an optical system (12) to direct a cutting section (15) of a laser beam (14) across a recess (6), parallel and spaced from a plane (8) of a skin engaging face (4), to cut hairs (10) which extend into the recess,
generating information indicative of the distance between plane of the skin engaging face and a distance by which a skin surface (5) has extended out of the plane of the skin engaging face into the recess,
a controller (30) determining the distance ($\overline{Y}_{skin}(z)$) by which the skin surface has extended out of the plane of the skin engaging face into the recess and activating a laser beam only if said distance by which the skin surface has extended out of the plane of the skin engaging face into the recess exceeds the distance between said plane of the skin engaging face and a predetermined laser beam activation threshold ($Y_{threshold}(z)$).

14. The method of cutting hair according to claim 13, further comprising the controller (30) deactivating the laser beam (14) when the distance ($\overline{Y}_{skin}(z)$) by which the skin surface (5) has extended out of the plane (8) of the skin engaging face (4) into the recess (6) exceeds a predetermined laser beam deactivation threshold.

15. The method of cutting hair according to claim 13 or claim 14, further comprising adjusting one or more elements of the optical system (12) in dependence on the information generated by the skin surface sensor (42) and/or the laser beam sensor (38) to adjust the distance between the predetermined laser beam deactivation threshold ($Y_{threshold}(z)$) and the plane (8) of the skin engaging face (4), such that the laser beam (14) is generated.

**Patentansprüche**

1. Laserrasiervorrichtung (1), umfassend
ein Gehäuse (2), das eine Hautauflagefläche (4) und eine Aussparung (6) aufweist, welche sich von der Hautauflagefläche in das Gehäuse hinein erstreckt,

wobei die Hautauflagefläche in einer Ebene (8) liegt oder selbige schneidet, welche sich über die Aussparung hinweg erstreckt, und derart gegen eine Hautoberfläche (5) positionierbar ist, dass die Hautoberfläche eine im Wesentlichen gewölbte Form annimmt, die sich in einer Richtung aus der Ebene der Hautauflagefläche heraus in die Aussparung hinein erstreckt,

ein optisches System (12), das dazu ausgebildet ist, einen Laserstrahl (14) über die Aussparung hinweg zu lenken, um Haare (10) zu schneiden, die sich von der Hautoberfläche innerhalb der Aussparung in einen Schneidabschnitt (15) des Laserstrahls hinein erstrecken,

einen Hautoberflächensensor (42), der dazu ausgebildet ist, ein Signal zu erzeugen, das einen Distanz ($\overline{Y}_{Haut}(z)$) angibt, um die sich eine Hautoberfläche aus der Ebene der Hautauflagefläche heraus in die Aussparung hinein erstreckt hat, und

eine Steuerung (30), die dazu ausgebildet ist, den Laserstrahl (14) nur dann zu aktivieren, wenn die Distanz, um die sich eine Hautoberfläche aus der Ebene der Hautauflagefläche heraus in die Aufnahme hinein erstreckt hat, eine Distanz zwischen der Ebene der Hautauflagefläche und einer vorbestimmten Laserstrahl-Aktivierungsschwelle ($Y_{Schwelle}(z)$) übersteigt.

2. Laserrasiervorrichtung (1) nach Anspruch 1, wobei die vorbestimmte Laserstrahl-Aktivierungsschwelle ($Y_{Schwelle}(z)$) zwischen der Ebene (8) der Hautauflagefläche (4) und einer Laserstrahl-Schneidhöhe ($Y_{Schneid}(z)$) in der Aussparung (6) liegt.

3. Laserrasiervorrichtung (1) nach Anspruch 2, wobei die vorbestimmte Laserstrahl-Aktivierungsschwelle ($Y_{Schwelle}(z)$) von der durchschnittlichen Distanz ($\mu_{Haut}$), um die sich die Hautoberfläche aus der Ebene (8) der Hautauflagefläche (4) heraus in die Aussparung (6) hinein erstreckt hat, vorzugsweise von sowohl der durchschnittlichen Distanz ($\mu_{Haut}$) als auch ihrer Standardabweichung ($\sigma_{Haut}$) abhängig ist.

4. Laserrasiervorrichtung (1) nach Anspruch 3, wobei die vorbestimmte Laserstrahl-Aktivierungsschwelle ($Y_{Schwelle}(z)$) größer als oder gleich $\mu_{Haut}$ - 2 * $\sigma_{Haut}$ und kleiner als oder gleich $\mu_{Haut}$ + 2 * $\sigma_{Haut}$ ab der Ebene (8) der Hautauflageoberfläche (4) ist.

5. Laserrasiervorrichtung (1) nach Anspruch 2, wobei die vorbestimmte Aktivierungsschwelle ($Y_{Schwelle}(z)$) ab der Ebene (8), die sich über die Aussparung (6) hinweg erstreckt, in die Aussparung (6) hinein im Bereich von 0 $\mu$m bis 300 $\mu$m beträgt.

6. Laserrasiervorrichtung (1) nach einem der vorstehenden Ansprüche, weiter einen Laserstrahlsensor (38) umfassend, der dazu ausgebildet ist, Informationen zu erzeugen, die die Position des Laserstrahls in der Aussparung relativ zur Ebene (8) der Hautauflagefläche (4) angeben.

7. Laserrasiervorrichtung (1) nach Anspruch 6, wobei die Steuerung (30) dazu ausgebildet ist, den Laserstrahl (14) in Abhängigkeit von den vom Hautoberflächensensor (42) und Laserstrahlsensor (38) erzeugten Informationen zu deaktivieren, wenn die Distanz ($\overline{Y}_{Haut}(z)$), um die sich die Hautoberfläche (5) aus der Ebene (8) der Hautauflagefläche (4) heraus in die Aussparung (6) hinein erstreckt hat, eine vorbestimmte Laserstrahl-Deaktivierungsschwelle ($Y_{Sicherheit}(z)$) übersteigt.

8. Laserrasiervorrichtung (1) nach einem der vorstehenden Ansprüche, wobei die Distanz zwischen der vorbestimmten Laserstrahl-Aktivierungsschwelle ($Y_{Schwelle}(z)$) und der Ebene (8) der Hautauflagefläche (4) kleiner ist als die Distanz zwischen einer vorbestimmten Laserstrahl-Deaktivierungsschwelle ($Y_{Sicherheit}(z)$) und der Ebene der Hautauflagefläche.

9. Laserrasiervorrichtung (1) nach einem der vorstehenden Ansprüche, weiter einen Aktor (45) umfassend, der dazu ausgebildet ist, auf das optische System (12) zu wirken, um die Position des Laserstrahls (14) relativ zu der Ebene (8), die sich über die Aussparung (6) hinweg erstreckt, anzupassen.

10. Laserrasiervorrichtung (1) nach einem der vorstehenden Ansprüche, weiter eine Benutzereingabe (35) umfassend, die dazu ausgebildet ist, es dem Benutzer zu ermöglichen, die gewünschte Nähestufe und/oder die vorbestimmte Laserstrahl-Aktivierungsschwelle ($Y_{Schwelle}(z)$) zu steuern.

11. Laserrasiervorrichtung (1) nach einem der vorstehenden Ansprüche, wobei der Laserstrahl (14) parallel zu und beabstandet von der sich über die Aussparung (6) hinweg erstreckenden Ebene (8), in welcher die Hautauflagefläche (8) liegt, über die Aussparung (6) hinweg gelenkt wird.

12. Laserrasiervorrichtung (1) nach einem der vorstehenden Ansprüche, wobei die Aussparung (6) in der Hautauflagefläche (4) in der Richtung senkrecht zur Richtung des Laserstrahls (14) größer als oder gleich 0,3 mm und kleiner als oder gleich 1,5 mm breit ist.

13. Verfahren zum Schneiden von Haar mit einer Laserrasiervorrichtung (1), umfassend
Betreiben eines optischen Systems (12) so, dass ein Schneidabschnitt (15) eines Laserstrahls (14) parallel und von einer Ebene (8) einer Hautauflagefläche (4) beabstandet über eine Aussparung (6) hinweg gelenkt wird, um Haare (10) zu schneiden, die sich in die Aussparung hinein erstrecken,
Erzeugen von Informationen, die die Distanz zwischen Ebene der Hautauflagefläche und einer Distanz angeben, um die sich eine Hautoberfläche (5) aus der Ebene der Hautauflagefläche heraus in die Aussparung hinein erstreckt hat,
Bestimmen, durch eine Steuerung (30), der Distanz ($\overline{Y}_{Haut}(z)$), um die sich die Hautoberfläche aus der Ebene der Hautauflagefläche heraus in die Aussparung hinein erstreckt hat, und Aktivieren eines Laserstrahls nur dann, wenn die Distanz, um die sich die Hautoberfläche aus der Ebene der Hautauflagefläche heraus in die Aussparung hinein erstreckt hat, die Distanz zwischen der Ebene der Hautauflagefläche und einer vorbestimmten Laserstrahl-Aktivierungsschwelle ($Y_{Schwelle}(z)$) übersteigt.

14. Verfahren zum Schneiden von Haar nach Anspruch 13, weiter das Deaktivieren des Laserstrahls (14) durch die Steuerung (30) umfassend, wenn die Distanz ($\overline{Y}_{Haut}(z)$), um die sich die Hautoberfläche (5) aus der Ebene (8) der Hautauflagefläche (4) heraus in die Aussparung (6) hinein erstreckt hat, eine vorbestimmte Laserstrahl-Deaktivierungsschwelle übersteigt.

15. Verfahren zum Schneiden von Haar nach Anspruch 13 oder Anspruch 14, weiter das Anpassen von einem oder mehreren Elementen des optischen Systems (12) in Abhängigkeit von den vom Hautoberflächensensor (42) und/oder dem Laserstrahlsensor (38) erzeugten Informationen umfassend, um die Distanz zwischen der vorbestimmten Laserstrahl-Deaktivierungsschwelle ($Y_{Schwelle}(z)$) und der Ebene (8) der Hautauflagefläche (4) derart anzupassen, dass der Laserstrahl (14) erzeugt wird.

**Revendications**

1. Dispositif de rasage laser (1) comprenant
un boîtier (2) présentant une face de prise de peau (4) et un évidement (6) s'étendant dans le boîtier depuis ladite face de prise de peau,

la face de prise de peau se trouvant dans ou croisant un plan (8) qui s'étend sur l'évidement et étant positionnable contre une surface de peau (5) de sorte que la surface de peau adopte une forme sensiblement en dôme s'étendant dans une direction hors du plan de la face de prise de peau dans l'évidement,
un système optique (12) configuré pour diriger un faisceau laser (14) sur ledit évidement pour couper des poils (10) s'étendant depuis ladite surface de peau dans l'évidement dans un section de coupe (15) du faisceau laser,
un capteur de surface de peau (42) configuré pour générer un signal indiquant une distance ($\overline{Y}_{peau}(z)$) de laquelle une surface de peau s'est étendue hors du plan de la face de prise de peau dans l'évidement, et
un élément de commande (30) configuré pour activer le faisceau laser (14) seulement si ladite distance de laquelle une surface de peau s'est étendue hors du plan de la face de prise de peau dans l'évidement excède une distance entre ledit plan de la face de prise de peau et un seuil d'activation de faisceau laser prédéterminé ($Y_{seuil}(z)$).

2. Dispositif de rasage laser (1) selon la revendication 1, dans lequel le seuil d'activation de faisceau laser prédéterminé ($Y_{seuil}(z)$) est entre ledit plan (8) de la face de prise de peau (4) et une hauteur de coupe de faisceau laser ($Y_{coupe}(z)$) dans l'évidement (6).

3. Dispositif de rasage laser (1) selon la revendication 2, dans lequel le seuil d'activation de faisceau laser prédéterminé ($Y_{seuil}(z)$) dépend de la distance moyenne ($\mu_{peau}$) de laquelle la surface de peau s'est étendue hors du plan (8) de la face de prise de peau (4) dans l'évidement (6), de préférence à la fois sur la distance moyenne ($\mu_{peau}$) et sa déviation standard ($\sigma_{peau}$).

**4.** Dispositif de rasage laser (1) selon la revendication 3, dans lequel le seuil d'activation de faisceau laser prédéterminé ($Y_{seuil}(z)$) est supérieur ou égal à $\mu_{peau}$ - 2* $\sigma_{peau}$ et inférieur ou égal à $\mu_{peau}$ + 2* $\sigma_{peau}$ du plan (8) de la face de prise de peau (4).

**5.** Dispositif de rasage laser (1) selon la revendication 2, dans lequel le seuil d'activation prédéterminé ($Y_{seuil}(z)$) est dans la plage de 0 $\mu$m à 300 $\mu$m du plan (8) qui s'étend sur l'évidement (6) dans l'évidement (6).

**6.** Dispositif de rasage laser (1) selon l'une quelconque des revendications précédentes, comprenant en outre un capteur de faisceau laser (38) configuré pour générer des informations indiquant la position du faisceau laser dans l'évidement relatif audit plan (8) de la face de prise de peau (4).

**7.** Dispositif de rasage laser (1) selon la revendication 6, dans lequel l'élément de commande (30) est configuré pour désactiver le faisceau laser (14) selon des informations générées par le capteur de surface de peau (42) et capteur de faisceau laser (38) lorsque la distance ($\overline{Y}_{peau}(z)$) de laquelle la surface de peau (5) s'est étendue hors du plan (8) de la face de prise de peau (4) dans l'évidement (6) excède un seuil de désactivation de faisceau laser prédéterminé ($Y_{sécurité}(z)$).

**8.** Dispositif de rasage laser (1) selon l'une quelconque des revendications précédentes, dans lequel la distance entre le seuil d'activation de faisceau laser prédéterminé ($Y_{seuil}(z)$) et le plan (8) de la face de prise de peau (4) est inférieure à la distance un seuil de désactivation de faisceau laser prédéterminé ($Y_{sécurité}(z)$) et le plan de la face de prise de peau.

**9.** Dispositif de rasage laser (1) selon l'une quelconque des revendications précédentes, comprenant en outre un actionneur (45) configuré pour agir sur le système optique (12) afin d'ajuster la position du faisceau laser (14) par rapport au plan (8) qui s'étend sur l'évidement (6).

**10.** Dispositif de rasage laser (1) selon l'une quelconque des revendications précédentes, comprenant en outre une entrée utilisateur (35) configurée pour permettre à l'utilisateur de commander le niveau de proximité souhaité et/ou le seuil d'activation de faisceau laser prédéterminé ($Y_{seuil}(z)$).

**11.** Dispositif de rasage laser (1) selon l'une quelconque des revendications précédentes, dans lequel le faisceau laser (14) est dirigé sur l'évidement (6) parallèle et espacé du plan (8) dans lequel la face de prise de peau (8) se trouve qui s'étend sur l'évidement (6).

**12.** Dispositif de rasage laser (1) selon l'une quelconque des revendications précédentes, dans lequel l'évidement (6) dans la face de prise de peau (4) est supérieur ou égal à 0,3 mm et inférieur ou égal à 1,5 mm de largeur dans la direction perpendiculaire à la direction du faisceau laser (14).

**13.** Procédé de coupe de poils avec un dispositif de rasage laser (1), comprenant
le fonctionnement d'un système optique (12) pour diriger une section de coupe (15) d'un faisceau laser (14) sur un évidement (6), parallèle et espacé d'un plan (8) d'une face de prise de peau (4), pour couper des poils (10) qui s'étendent dans l'évidement,
la génération des informations indiquant la distance entre le plan de la face de prise de peau et une distance de laquelle une surface de peau (5) s'est étendue hors du plan de la face de prise de peau dans l'évidement,
un élément de commande (30) déterminant la distance ($\overline{Y}_{peau}(z)$) de laquelle la surface de peau s'est étendue hors du plan de la face de prise de peau dans l'évidement et activant un faisceau laser seulement si ladite distance de laquelle la surface de peau s'est étendue hors du plan de la face de prise de peau dans l'évidement excède la distance entre ledit plan de la face de prise de peau et un seuil d'activation de faisceau laser prédéterminé ($Y_{seuil}(z)$).

**14.** Procédé de coupe de poils selon la revendication 13, comprenant en outre l'élément de commande (30) désactivant le faisceau laser (14) lorsque la distance ($\overline{Y}_{peau}(z)$) de laquelle la surface de peau (5) s'est étendue hors du plan (8) de la face de prise de peau (4) dans l'élément (6) excède un seuil de désactivation de faisceau laser prédéterminé.

**15.** Procédé de coupe de poils selon la revendication 13 ou 14, comprenant en outre l'ajustement d'un ou plusieurs éléments du système optique (12) selon les informations générées par le capteur de surface de peau (42) et/ou le capteur de faisceau laser (38) pour ajuster la distance entre le seuil de désactivation de faisceau laser prédéterminé ($Y_{seuil}(z)$) et le plan (8) de la face de prise de peau (4) de sorte que le faisceau laser (14) soit généré.

Fig. 1

Fig. 2

Fig. 3

Fig. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014139968 A1